# EUROPEAN PATENT SPECIFICATION

(11) **EP 2 540 276 B1**
(45) Date of publication and mention of the grant of the patent: **16.03.2016**
(21) Application number: 12180625.1
(22) Date of filing: 24.05.2007
(51) Int. Cl.: A61J 1/20, B65B 3/00, B65D 45/32, B65D 55/08

(54) **Method of assembling a reconstitution device**
Verfahren zur Herstellung einer Rekonstitutionsvorrichtung
Méthode d'assemblage d'un dispositif de reconstitution

(30) Priority: 25.05.2006 US 803187 P
(43) Date of publication of application: 02.01.2013
(62) Divisional of application: 07762316.3
(73) Proprietor: Bayer Healthcare LLC, Whippany, NJ 07981-0915 (US)
(72) Inventor: Tuckwell, Jonathan David, Cambridge CB4 1YB, Cambridgeshire (GB); Dyer, Robert, Cambridge 02138, MA Massachusetts (US); Kivlin, Robert Owen, Norfolk PE31 6BH, Norfolk (GB); Palmer-Felgate, John Paul, Winchester SO22 4JY, Hampshire (GB); Avery, Matthew Burgess, Palo Alto, CA 94306 (US); Skinner, Kevin, George, Oakland, CA 94619-3727 (US); Kadamus, Chris, Jamaica Plain, MA 02130, MA Massachusetts (US); Wood, Lee, South Wales CF31 4QP, South Wales (GB); Schwan, Peter, 51373 Leverkusen (DE); Arlett, Ben, Bristol BS7 9DJ (US)
(74) Representative: BIP Patents

(56) References cited:
- FR-A- 2 753 624

## Description

### Field of the Invention

The present application relates generally to reconstitution devices. More particularly, the application relates to an improved reconstitution device for connecting a closed receptacle and a container, such as a syringe.

### Description of Related Art

In the domain of drug-packaging, it is known to store a component of a medicinal preparation, such as for example its active ingredient, in a recipient closed by a stopper of relatively non-rigid material, for example of elastomer. A liquid may be introduced into this recipient after perforation of the stopper in order to dissolve the component contained in the recipient or place it in suspension, with a view to obtaining a medicinal preparation in liquid form ready to be administered to the patient.

Traditional devices include a base adapted to cover the neck of the recipient and extending in a flange forming an inner bore while a plunger is adapted to slide in the bore, between a position disengaged with respect to the stopper and an engaged position in which a hollow needle borne by the plunger traverses this stopper. The displacement of the plunger from its disengaged position towards its engaged position is effected manually by an operator.

FR 2753624 discloses a connecting device between a first receptacle and a second receptacle comprising a muff joint. The connecting device comprises an apparatus for perforating a stopper, including a faucet and a filtering chamber isolated from outside by a filter, two independent channels being provided in the perforating apparatus for communicating the inside of the first receptacle with the faucet and the filtering chamber respectively. The device further comprising an element for displacing with guidance the perforating apparatus, an element for fastening the skirt on the neck, a plunger mounted in the internal bore on which the perforating apparatus are fixed, for sliding by simple pressure, and an element for definitively stopping the plunger.
The skirt is obtained in one piece with the fastening means, for example made of plastic material, may extend upwardly, beyond the free end of the faucet, in order to prevent an accidental actuation of the plunger by the user's fingers. Furthermore, it is provided with a capsule forming fastening means, capable of clipping with respect to and beneath the annular rim of the receptacle, in contact with the neck, and this thanks to a radial elasticity enabling it to return the circumferential lower edge into centripetal position. In practice, this fastening capsule is constituted by a plurality of fastening teeth, together forming the capsule defined hereinabove, and each presenting the radial elasticity mentioned above. In the fastened position the internal bore opens out on the stopper and more particularly its upper part accessible to the perforating end of the perforating means.
In the device disclosed in FR 2753624 the clipping of the connecting device to the receptacle is performed by fastening means having a certain radial elasticity allowing it to return to the fastening position after have been bended during the clipping procedure. However, the elasticity of materials might degrade over the time especially due to external influences. Accordingly, it is desirable to use fastening means being more resistant to degradation of elasticity than those of the known devices.

### SUMMARY

The present invention is directed to a method of assembling a drug reconstitution device as claimed in claim 1 and claim 2.

The present invention helps to solve the shortcomings of the prior art.

These as well as other aspects and advantages will become apparent to those of ordinary skill in the art by treading the following detailed description, with reference where appropriate to the accompanying drawings. Further, it should be understood that the embodiments described in this summary and elsewhere are intended to illustrate the invention by way of example only.

### BRIEF DESCRIPTION OF THE DRAWINGS

Exemplary embodiments of the invention are described herein with reference to the drawings, in which:
Figure 1 is an expanded view of a reconstitution device of the present invention;
Figure 2 is a cross-sectional view of the reconstitution device of Figure 1;
Figure 3 is a cross-sectional view of a second embodiment of a reconstitution device of the present invention;
Figure 4 is a perspective view of the reconstitution device of Figure 3 including a top.

### DETAILED DESCRIPTION

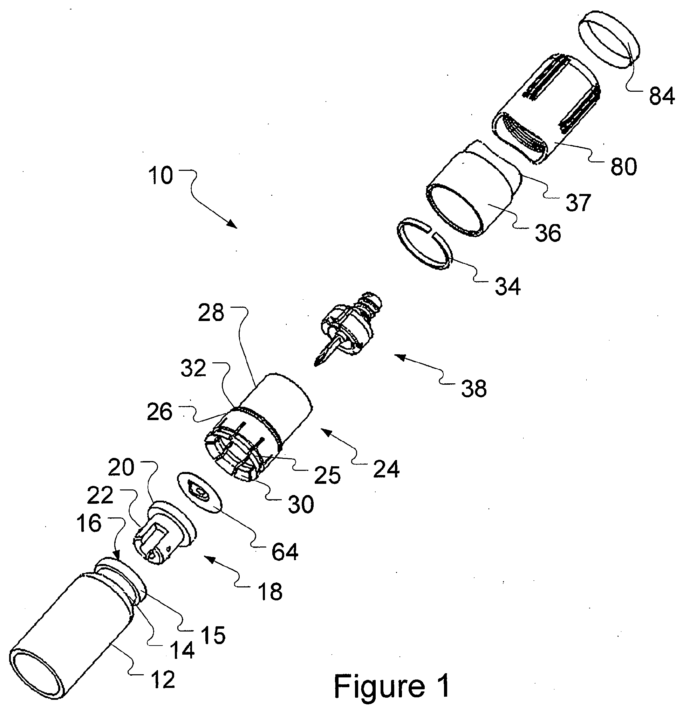

Figure 1 depicts a reconstitution device 10. The device 10 includes a receptacle 12 for storing a first component of a pharmaceutical preparation (not shown), such as its active ingredient, for example. The receptacle 12 includes an opening 16 surrounded or partially surrounded by a neck 14. The neck 14 also includes a lip 15. The opening.16 in the neck 14 allows for a second component (not shown), such as a liquid, to be introduced into the receptacle 12 and mix with the first component. A stopper 18 is positioned in the opening 16 of the neck 14 to block access to the receptacle 12. The stopper 18 may be made of a relatively non-rigid material, such as elastomer. The stopper 18 may include a top portion 20 located against the lip 15, and a bottom portion 22 located within the opening of the neck 14. The top portion 20 may be capable of being perforated, thereby allowing access to the receptacle 12.

The device 10 further includes a cap 24 secured to the neck 14 of the receptacle 12. The cap 24 has a first end 26, which is secured to the neck 14 of the receptacle 12, and a second end 28 located opposite the first end 26. The first end 26 of the cap 24 may surround at least a portion of the stopper 18. The first end 26 of the cap 24 may include one or more flexible legs 30 so the cap 24 can expand to fit over the lip 15 of the neck 14 during the manufacturing process, and then contract to securely mate with the neck 14. The cap 24 may further include a step portion 32 located between the first end 26 and the second end 28, thereby separating the first end 26 from the second end 28. The first end 26 of the cap 24 may have a larger diameter than the second end 28 of the cap 24.

The first end 26 of the cap 24 is secured to the receptacle 12 by a C-clip 34, which is positioned in an indentation 25 in the first end 26 of the cap 24. Therefore, the C-clip 34 is not located directly on the neck 14 of the receptacle 12 and no scratching occurs. The C-clip 34 may be made of a material not subject to heat degradation, such as metal for example, so that the device 10 may be resistant to heat tampering.

The first end 26 of the cap may be surrounded by a sleeve 36 for protection. The sleeve 36 is prevented from sliding off the cap 24 by the step portion 32. The sleeve 36 may include a wave-shaped part 37 to facilitate opening by a user (not shown). The sleeve 36 may further include a textured surface to facilitate gripping of the device 10 by a user.

Assembly of the first end 26 of the cap 24 to the receptacle 12 may be carried out using various methods. For example, an assembly method may include initially inserting the C-clip 34 into the indentation 25, and then sliding the cap 24 over the stopper 18 and the lip 15. As another example, an assembly method may include initially placing the C-clip 34 onto the second end 28 of the cap 24, then sliding the sleeve 36 over the second end 28 in such a way that the sleeve 36 contacts the C-clip 34 and pushes the C-clip 34 into the indentation 25, and then sliding the cap 24 over the stopper 18 and the lip 15. In accordance with these exemplary methods, as the cap 24 is slid over the stopper 18 and the lip 15, the opening in the C-clip 34 allows the flexible legs 30 to expand as the flexible legs 30 pass over the lip 15, and the flexible legs 30 may contract after passing over the lip 15 to secure the cap to the receptacle 12.

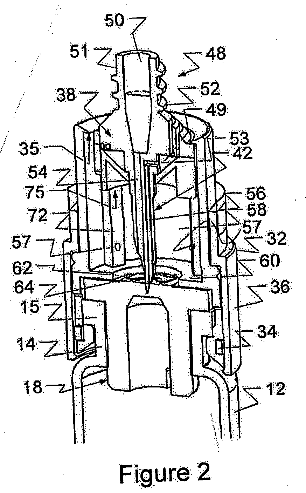

Referring to Figure 2, the shaft 42 of the plunger 38 may include a first longitudinal channel 54. The first longitudinal channel 54 establishes communication between the receptacle 12 and the inner bore 50 of the male element 48. The first channel 54 may allow fluid to pass through the shaft 42 of the plunger 38 and into the receptacle 12.

The shaft 42 of the plunger 38 may include a second longitudinal channel 56. The end of the second channel 56 located opposite the pointed end 44 of the shaft 42 may interface to an air channel return 53. The air channel return 53 interfaces to a cavity 49. The cavity 49 is formed by joining the first portion 39 of the plunger 38 to the second portion 40 of the plunger 38. The second channel 56 functions as an air path to allow air to travel out of the receptacle 12 through the shaft 42 of the plunger 38, into the air channel return 53, through the filter 46, and then through the vent 47 located on the top surface 41 of the plunger 38. The air exiting the vent 47 is vented to the atmosphere surrounding the device 10.

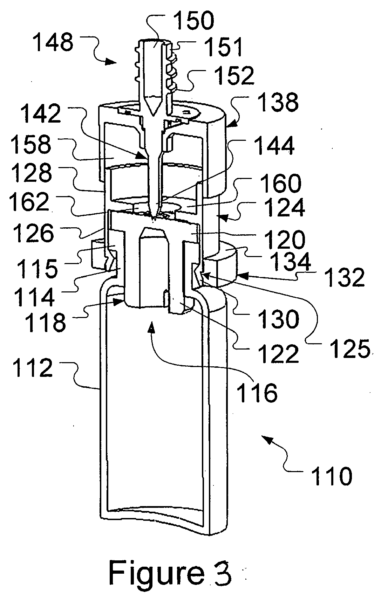

The cap 24 may further include an inner bore 58 having an annular plate 60 with an aperture 62. The aperture 62 is coaxially aligned with the shaft 42 of the plunger 38. The plunger 38 may be configured to slide along the inner bore 58 of the cap 24, or alternatively, the plunger 38 may be configured to slide along a outer surface of the cap, as shown in Figure 3, which will be described below. When the plunger 38 is pushed downward toward the receptacle 12, the shaft 42 moves downwardly through the aperture 62 to pierce the top portion 20 of the stopper 18.

Referring to Figure 3, a second embodiment 110 of the reconstitution device of the present invention is shown. The device 110 includes a receptacle 112 for storing a first component of a pharmaceutical preparation (not shown), such as its active ingredient, for example. The receptacle 12 includes an opening 116 surrounded or partially surrounded by a neck 114. The neck 114 also includes a lip 115. The opening 116 in the neck 114 allows for a second component, such as a liquid (not shown), to be introduced into the receptacle 112 and mix with the first component. A stopper 118 is positioned in the opening 116 of the neck 114 to block access to the receptacle 112. The stopper 118 may be made of a relatively non-rigid material, such as elastomer. The stopper 118 may include a top portion 120 located against the lip 115, and a bottom portion 122 located within the opening 116 of the neck 114. The top portion 120 may be capable of being perforated, to allow access to the receptacle 112.

The device 110 further includes a cap 124 secured to the neck 114 of the receptacle 112. The cap 124 has a first end 126, which is secured to the neck 114 of the receptacle 112, and a second end 128 located opposite the first end 126. The first end 126 of the cap 124 may surround at least a portion of the stopper 118. The first end 126 of the cap 124 may include a protrusion 130 for securing the first end 126 to the lip 115 of the receptacle 112.

The first end 126 of the cap 124 may further be secured to the receptacle 112 by a crimp ring 132, which may be positioned in an indentation 125 in the first end 126 of the cap 124. The crimp ring 132 extends around only a portion of the circumference of the first end 126 of the cap 124. The crimp ring 132 may be made of metal or a polymer with low creep sensitivity. The crimp ring 132 may further include an upper surface 134 for attaching to a top 180, which will be described below.

The device 110 may include a plunger 138 located at the second end 128 of the cap 124. The plunger 138 may include a shaft 142 extending in a direction towards the stopper 118. The shaft 142 may include a pointed end 144 for piercing the top portion 120 of the stopper 118, thereby allowing the shaft 142 access to the receptacle 112. The shaft 142 may further include a filtering mechanism (not shown) similar in structure and function to the filtering mechanism disclosed above with respect to device 10.

The plunger 138 may also include a male element 148 extending in a direction opposite the shaft 142. The male element 148 may be configured to receive a second receptacle (not shown), such as a syringe, for example. The male element 148 may include an inner bore 150 and an outer surface 151. The outer surface 151 may include a thread 152 for mating with the second receptacle.

The shaft 142 of the plunger 138 may also include first and second longitudinal channels (not shown) which establish communication between the receptacle 112 and the inner bore 150 of the male element 148. The longitudinal channels are similar in structure and function to the longitudinal channels described above with respect to device 10. The plunger 138 may further be configured to slide along the outer circumference of the cap 124, as shown in Figure 3.

The cap 124 may further include an inner bore 158 having an annular plate 160 with an aperture 162. The aperture 162 is coaxially aligned with the shaft 142 of the plunger 138. When the plunger 138 moves downward toward the receptacle 112, the shaft 142 moves downwardly through the aperture 162 to pierce the top portion 120 of the stopper 118.

The device 110 may include a mechanism that prevents upward movement of the plunger 138 after downward movement of the plunger 138 towards the stopper 118 has occurred. As an example, the device 110 may include a locking mechanism such as the locking mechanism 64 described above. The locking mechanism may be used to retain the plunger 138 to the device 110.

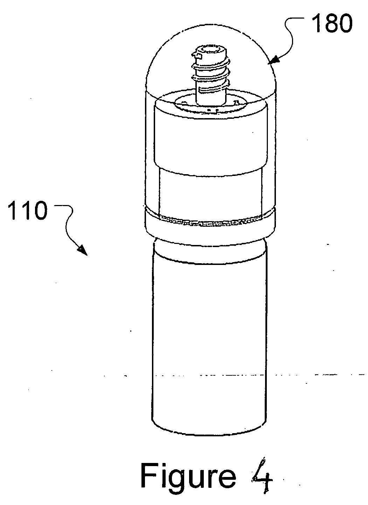

The device 110 may include a top 180, as shown in Figure 4, which fits over the second end 128 of the cap 124 and the plunger 138, and attaches to the crimp ring 132. The top 180 protects the device 110.

Moreover, the top 180 and the crimp ring 132 may be formed as a single piece (i.e., a top and crimp ring combination (not shown)). To accommodate the top and crimp ring combination, the cap 124 may have a first end and a second end, and the first end may be larger in diameter than the second end (similar to the first end 26 of the cap 24 and the second end 28 of the cap 24 shown in Figure 1). In this way, the top and crimp ring combination may slide over the plunger 138 so as to allow the cap 124 to be secured to the receptacle 112. To use the device 110 with the top and crimp ring combination, the top may be broken off (e.g., by twisting the top) and the crimp ring continues to secure the cap 124 to the receptacle 112.

Additionally, a tamper-proof mechanism (not shown) may be located on the device 110 to indicate to a user whether the device has been used. The tamper-proof mechanism may comprise any type of indicator, such as a seal, a holographic label, or a tab, for example.

In operation, the device 110 is in a disengaged position, that is, the shaft 142 of the plunger 138 is not piercing the stopper. Once the top 180 is removed from the device 110, the tamper-proof mechanism will be broken. A user may then attach a second receptacle, such as a syringe, to the receptacle 112. The plunger 138 may then be pushed in a downward direction toward the stopper 118 into an engaged position. The shaft 142 of the plunger 138 may pierce the stopper 118, allowing access to the opening 116 of the receptacle 112. The contents of the second receptacle may then be introduced into the receptacle 112 to mix with the component. The mixed contents may then be pulled back into the second receptacle. A needle (not shown) may then be secured to the second receptacle, and the complete and active drug may be administered to a patient.

While certain features and embodiments of the present invention have been described in detail herein, it is to be understood that the invention encompasses all modifications and enhancements within the scope of the following claims.

## Claims

1. A method of assembling a drug reconstitution device (10) comprising:
providing a receptacle (12) having a lip (15) surrounding an opening (16);
inserting a stopper (18) into the opening (16) of the receptacle (12);
providing a cap (24) having a first end (26) and including flexible legs (30), and a second end (28)
opposite the first end (26);
**characterized in** the further steps of
inserting a C-clip (34) into an indentation (25) at the first end (26) of the cap (24);
sliding the first end (26) of the cap (24) over the stopper (18) and the lip (15) of the receptacle (12),
wherein the C-clip (34) allows the flexible legs (30) to expand as the flexible legs (30) pass over the lip (15), and allows the flexible legs (30) to contract after passing over the lip (15) to secure the cap (24) to the receptacle (12).

2. The method of claim 1, wherein the first end (26) of the cap is surrounded by a sleeve (36) and the step of inserting the C-clip (34) into an indentation (25) at the first end (26) of the cap (24) comprises
placing the C-clip (34) onto the second end (28) of the cap (24);
sliding the sleeve (36) over the second end (28) in such a way that the sleeve (36) contacts the C-clip (34) and pushes the C-clip (34) into the indentation (25).

## Patentansprüche

1. Verfahren zum Zusammensetzen einer Arzneimittel-Rekonstituierungsvorrichtung (10), umfassend:
Bereitstellen eines Behälters (12) mit einer Lippe (15), die eine Öffnung (16) umgibt;
Einführen eines Stopfens (18) in die Öffnung (16) des Behälters (12);
Bereitstellen einer Kappe (24) mit einem ersten Ende (26), das flexible Beine (30) aufweist, und einem zweiten Ende (28) gegenüber dem ersten Ende (26);
**gekennzeichnet durch** die weiteren Schritte
Einführen eines C-Clips (34) in eine Vertiefung (25) an dem ersten Ende (26) der Kappe (24);
Schieben des ersten Endes (26) der Kappe (24) über den Stopfen (18) und die Lippe (15) des Behälters (12),
wobei der C-Clip (34) den flexiblen Beinen (30) erlaubt, zu expandieren, wenn die flexiblen Beine (30) über die Lippe (15) passieren, und den flexiblen Beinen (30) erlaubt, zu kontrahieren, nachdem sie über die Lippe (15) passiert sind, um die Kappe (24) an dem Behälter (12) zu befestigen.

2. Verfahren gemäß Anspruch 1, wobei das erste Ende (26) der Kappe von einer Hülse (36) umgeben ist und der Schritt des Einführens des C-Clips (34) in eine Vertiefung (25) an dem ersten Ende (26) der Kappe (24) umfasst:
Anordnen des C-Clips (34) auf dem zweiten Ende (28) der Kappe (24);
Schieben der Hülse (36) über das zweite Ende (28) auf eine solche Weise, dass die Hülse (36) in Kontakt mit dem C-Clip (34) kommt und den C-Clip (34) in die Vertiefung (25) drückt.

## Revendications

1. Procédé d'assemblage d'un dispositif de reconstitution de médicaments (10), comprenant :
fournir un réceptacle (12) présentant une lèvre (15) entourant une ouverture (16) ;
insérer un bouchon (18) dans l'ouverture (16) du réceptacle (12) ;
fournir un capuchon (24) ayant une première extrémité (26) et comportant des pattes flexibles (30) et une deuxième extrémité (28) opposée à la première extrémité (26) ;
**caractérisé par** les étapes supplémentaires consistant à
insérer un circlip en forme de C (34) dans une indentation (25) au niveau de la première extrémité (26) du capuchon (24) ;
faire coulisser la première extrémité (26) du capuchon (24) par-dessus le bouchon (18) et la lèvre (15) du réceptacle (12),
le circlip en forme de C (34) permettant aux pattes flexibles (30) de s'écarter à mesure que les pattes flexibles (30) passent par-dessus la lèvre (15), et
permettant aux pattes flexibles (30) de se contracter une fois qu'elles sont passées par-dessus la lèvre (15) de manière à fixer le capuchon (24) au réceptacle (12).

2. Procédé selon la revendication 1, dans lequel la première extrémité (26) du capuchon est entourée par un manchon (36) et l'étape consistant à insérer le circlip en forme de C (34) dans une indentation (25) au niveau de la première extrémité (26) du capuchon (24) comprend
placer le circlip en forme de C (34) sur la deuxième extrémité (28) du capuchon (24) ;
faire coulisser le manchon (36) par-dessus la deuxième extrémité (28) de telle sorte que le manchon (36) vienne en contact avec le circlip en forme de C (34) et pousse le circlip en forme de C (34) dans l'indentation (25).
